# EUROPÄISCHE PATENTSCHRIFT

(11) **EP 0 349 940 B1**
(45) Veröffentlichungstag und Bekanntmachung des Hinweises auf die Patenterteilung: **13.05.1998**
(21) Anmeldenummer: 89112062.8
(22) Anmeldetag: 01.07.1989
(51) Int. Cl.: C07D 207/40, C07D 211/88, C07D 207/44, C07D 209/48, C07D 223/10, C11D 3/395

(54) **Phthalimidoperoxihexansäure, Verfahren zu deren Herstellung und deren Verwendung**
Phthalimidohexanperacid, process for preparing the same and use thereof
Phthalimidohexanpéracide, procédé pour sa préparation et son application

(30) Priorität: 08.07.1988 DE 3823172
(43) Veröffentlichungstag der Anmeldung: 10.01.1990
(73) Patentinhaber: Clariant GmbH, 65929 Frankfurt am Main (DE)
(72) Erfinder: Gethöffer, Hanspeter, Dr., D-6000 Frankfurt am Main 71 (DE); Reinhardt, Gerd, Dr., D-6233 Kelkheim (Taunus) (DE)

(56) Entgegenhaltungen:
- EP-A- 0 170 386
- EP-A- 0 260 134
- EP-A- 0 325 288
- EP-A- 0 325 289
- JOURNAL OF THE CHEMICAL SOCIETY 1962, Seiten 3821,3822, London, GB; K. BALENOVIC et al.: "Preparation of some peroxy-acids derived from optically active amino-acids"
- CHEMISCHE BERICHTE Band 101, Nr 1, 1968,Seiten 3957-3962; C. RUECHARDT et al.:"Alpha-Acylamino-percarbonsäure-tert.-butylester" * Seite 3958, Tabelle I *

## Beschreibung

Anorganische Persalze sind seit langem als Bleichzusätze in Waschmitteln bekannt. Da sie ihre optimale Bleichkraft jedoch erst bei Temperaturen oberhalb 60°C entfalten, werden zu ihrer Aktivierung eine Reihe von organischen Verbindungen beschrieben, die während des Waschprozesses mit Wasserstoffperoxid unter Freisetzung einer Peroxicarbonsäure reagieren, welche bereits bei 40 - 60°C bleichend wirkt. Eine Übersicht zahlreicher bekannter Perborataktivatoren wie N-Acylverbindungen (Tetraacetylethylendiamin, Tetraacetylmethylendiamin, Tetraacetylglycoluril) oder aktivierte Ester (Pentaacetylglucose, Acetoxibenzolsulfonat-Natrium, Benzoyloxibenzolsulfonat-Natrium) ist z.B. in US 4, 248,928 gegeben.

Daneben werden in neuerer Zeit eine Reihe organischer Peroxicarbonsäuren als Bleichsysteme für Waschmittel beschrieben. Neben bereits kommerziell erhältlichen Peroxicarbonsäuren wie Dodecandipercarbonsäure (EP 127 782) und Monoperoxiphthalsäure (EP 27 693) sind Perbernsteinsäure (DE 34 38 529), Perglutarsäure (DE 35 39 036) und Sulfoperbenzoesäure (EP 124 969) beschrieben. Problematisch an diesen Peroxicarbonsäuren ist jedoch ihre geringe Lagerstabilität, die zum Teil erst durch besondere physikalische oder chemische Stabilisierung gewährleistet wird. Insbesondere hat sich hier die Herstellung von Magnesium-Salzen (EP 105 689) oder ein Zusatz von Phosphanoxid/Natriumsulfat (DE 33 20 497) bewährt. Nach EP 170 386 werden organische Peroxicarbonsäuren auch durch eine zusätzliche Amidgruppe im Molekül stabilisiert. Beispiele dafür sind N-Decanoyl-6-aminoperoxicapronsäure oder 3-(N-Nonyl-carbamoyl)perpropionsäure. Die Lagerstabilisät dieser Verbindungen kann durch Überführung in das Magnesiumsalz und durch Zugabe von Boraten zusätzlich gesteigert werden (US 4,686,063). Die bisher beschriebenen freien Peroxicarbonsäuren zeigen jedoch bereits unter relativ milden Lagerbedingungen eine deutliche Abnahme des Aktivsauerstoffs. Dies ist auch, wie eigene Versuche zeigen, bei den bereits in J. Chem. Soc. 1982, 3821 und Chem. Ind. 1961, 469 beschriebenen Imidopercarbonsäuren der Fall. Es besteht daher weiterhin ein großes Interesse an lagerstabilen organischen Peroxicarbonsäuren mit hoher Bleicheffizienz, die auf einfachem und sicherem Wege bequem zugänglich sind.

Gegenstand der Erfindung ist ω-Phthalimidoperoxihexansäure der Formel

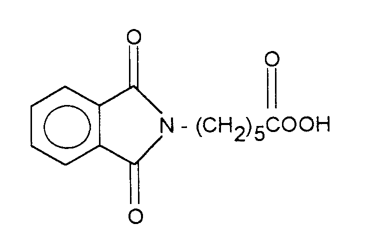

Die Herstellung dieser Verbindung erfolgt durch die Schritte:
-a- Synthese der Imidocarbonsäure
-b- Oxidation zur Percarbonsäure
-c- Isolierung der Imidoperoxicarbonsäure.

Im folgenden werden die einzelnen Schritte näher erläutert. Die Herstellung der Imidocarbonsäure im Schritt -a- kann in an sich bekannter Weise durch Umsetzung von Phthalsäureanhydrid mit ω-Aminocapronsäure erfolgen (s. Houben-Weyl, Methoden der Organischen Chemie, XI/2, S. 17).

Die Imidocarbonsäure kann besonders kostengünstig auch aus ε-Caprolactam hergestellt werden. Hierzu wird das Lactam mit dem Anhydrid in Gegenwart von Wasser unter Druck 2 bis 20 h, vorzugsweise 5 bis 10 h bei einer Temperatur von 100 bis 250°C, vorzugsweise 120 bis 200°C, unter Inertgasatmosphäre in einem geeigneten Reaktionsgefäß umgesetzt. Der Überdruck kann 1 bis 30 bar, vorzugsweise 2 bis 5 bar betragen.

Die Umwandlung der im Schritt -a- erhaltenen Imidocarbonsäure zur Imidopercarbonsäure erfolgt durch Umsetzung mit einem Oxidationsgemisch aus Wasserstoffperoxid und einer starken Säure. Wasserstoffperoxid kommt als 30 bis 95 prozentige, vorzugsweise 35 bis 50 prozentige wäßrige Lösung zum Einsatz. Geeignete saure Katalysatoren sind neben Schwefelsäure Methansulfonsäure oder ein saurer Ionenaustauscher. Schwefelsäure wird als 50 bis 96 prozentige, vorzugsweise 75 bis 96 prozentige wäßrige Lösung verwendet.

Wasserstoffperoxid wird in 1- bis 20-fachem, vorzugsweise 1.5- bis 4-fachem molarem Überschuß verwendet. Es hat sich ferner als günstig erwiesen, die Wasserstoffperoxidzugabe portionsweise vorzunehmen. Im allgemeinen wird ein 2- bis 5-facher Überschuß an Schwefelsäure - bezogen auf die Imidocarbonsäure - eingesetzt. Die Reaktionstemperatur liegt zwischen 5 und 50°C, vorzugsweise zwischen 15 und 45°C.

Da die beanspruchte Imidoperoxicarbonsäure aus dem Reaktionsgemisch ausfällt, kann sie in Schritt -c- in einfacher Weise durch Filtration oder Zentrifugation isoliert werden. Der Ausfällungsprozess kann durch Zugaben von Wasser beschleunigt und vervollständigt werden. Es ist auch möglich, durch Extraktion mit einem organischen Lösemittel die Imidoperoxicarbonsäure abzutrennen.

Die erfindungsgemäße Imidoperoxicarbonsäure ist fest, nahezu geruchlos, besitzt einen niedrigen Dampfdruck und ist von ausgezeichneter thermischer Stabilität. Sie kann als Lösung, Pulver oder in verarbeiteter Form allein oder in Kombination mit weiteren Stoffen zu Bleich-, Oxidations- oder Desinfektionszwecken eingesetzt werden.

Bevorzugt wird sie als Bleichmittel in festen oder flüssigen Wasch- und Reinigungsmitteln eingesetzt, da ihre bleichende und desinfizierende Wirkung bereits in einem weiten Temperaturbereich unterhalb von 60°C voll wirksam wird.

Zur Einarbeitung in pulverförmige Waschmittel eignet sie sich insbesondere in granulierter, extrudierter, tablettierter oder agglomerierter Form. Als Zusätze bei dieser Verarbeitungsweise sind an sich bekannte Hilfsmittel wie Borsäure, Sulfate, Phosphate, Carbonate, Zeolithe, Carboximethylcellulose usw. und filmbildende Stoffe wie Fettsäuren, Fettsäureamide oder -ester, Fettalkoholpolyglykolether oder Polyethylenglykole möglich.

Die erfindungsgemäße Verbindung erweist sich als überlegen im Vergleich zu dem bekannten Bleichsystem Perborat/TAED (Tetraacetylethylendiamin). Während Bleichsysteme auf Perborat-Basis bei Blutanschmutzungen zur Fixierung des Blutes auf dem Gewebe neigen und damit seine Auswaschbarkeit drastisch herabsetzen, wird dieser antagonistische Effekt beim Einsatz der hier beschriebenen erfindungsgemäßen Peroxicarbonsäure nicht beobachtet.

Bei Verwendung äquimolarer Mengen an Aktivsauerstoff ist die erfindungsgemäße Verbindung in ihrer Bleicheffizient an Tee- und Rotweinanschmutzungen den bisher beschriebenen Peroxicarbonsäuren und -dicarbonsäuren überlegen. Vorteilhaft ist weiterhin eine signifikante Öllöslichkeit der hier beschrieben Verbindung, wodurch insbesondere hydrophobe ölhaltige Anschmutzungen wie Grillöl oder Spaghettisauce gut gebleicht werden.

### Beispiel

### a) ω-Phthalimidohexansäure

113,2 g (1 Mol) ε-Caprolactam, 18 g Wasser und 148,1 g (1 Mol) Phthalsäureanhydrid werden in einem Autoklaven 5 h bei 160°C und 3 bar Stickstoff zur Reaktion gebracht. Die Schmelze wird anschließend in eine Porzellanschale gegossen.
Ausbeute: 261 g (99,8 %), weiße Kristalle
Fp: 104 - 105°C

### b) ω-Phthalimidoperoxihexansäure

653,2 g (2,5 Mol) ω-Phthalimidohexansäure werden wie folgt oxidiert:

Die Imidocarbonsäure wird in der 2- is 2,5-fachen Menge Schwefelsäure gelöst und tropfenweise mit 2,5 Äquivalenten Wasserstoffperoxid (35 - 50 prozentig) unter Eiskühlung 50 versetzt, daß die Innentemperatur zwischen 40 und 45°C gehalten werden kann. Nach erfolgter Zugabe wird auf 25 - 30°C gekühlt, mit Wasser verdünnt und die ausgefallene Persäure abgesaugt. Der Filterkuchen wird mit Wasser gewaschen und im Vakuumtrockenschrank bei 35°C getrocknet.
Ausbeute: 636,6 g (91,8 %), weiße Kristalle
Aktivsauerstoffgehalt: 5,4 % (93,6 %)
Fp: 89 - 90°C

### Waschversuche im Launder-O-Meter

Die Versuche wurden bei 40 und 60°C im Launder-O-Meter unter Verwendung der Testanschmutzungen Tee auf WFK-Baumwolle, Rotwein auf EMPA-Baumwolle und Standard-Anschmutzung auf WFK-Baumwolle durchgeführt. Die Bleichsysteme wurden so dosiert, daß jeweils 25 mg/l Aktivsauerstoff in der Waschflotte resultierten. Als Waschmittel wurden jeweils 1,5 g/l IEC-Waschmittel eingesetzt. Die Waschzeit betrug 30 min. Die Bleichwirkung wurde als Remissionszunahme an den verschiedenen Testgeweben bestimmt. Die Auswertung erfolgt in gewohnter Weise.

| Bleichsystem | Remissionswerte | | | | | |
|---|---|---|---|---|---|---|
| | Standard | | Tee | | Rotwein | |
| | 40°C | 60°C | 40°C | 60°C | 40°C | 60°C |
| Perborat | 49,5 | 59,4 | 58,4 | 62,8 | 55,5 | 56,9 |
| Perborat/TAED | 48,3 | 63,6 | 66,9 | 67,4 | 59,8 | 60,0 |
| PAP | 49,0 | 64,9 | 72,2 | 75,0 | 67,7 | 73,1 |

Die Ergebnisse zeigen bei äquimolarem Einsatz der Bleichsysteme eine deutlich bessere Bleichleistung der erfindungsgemäßen Persäure PAP (ω-Phthalimidoperoxihexansäure) gegenüber dem bisher üblichen Bleichsystem Perborat/TAED (Tetraacetylethylendiamin).

### Waschversuche im Launder-O-Meter

Die Versuche wurden analog wie zuvor beschrieben durchgeführt. Als Bleichsysteme wurden die Peroxicarbonsäuren DPDDA (Diperoxidodecandisäure), PMP (Monoperphthalsäure), NAPSA (Nonylmonoamidoperbernsteinsäure) gegen PAP ausgeprüft.

| Peroxisäuren | Remissionswerte | | | |
|---|---|---|---|---|
| | Tee | | Rotwein | |
| | 38°C | 60°C | 38°C | 60°C |
| DPDDA | 69,6 | 73,6 | 65,5 | 71,8 |
| PMP | 66,8 | 71,1 | 61,1 | 65,4 |
| NAPSA | 68,1 | 72,2 | 63,7 | 68,6 |
| PAP | 68,4 | 77,1 | 67,2 | 74,3 |

### Waschversuche in der Waschmaschine

Die Versuche wurden in einer Miele Waschmaschine (Automatic W432) mit dem 40°-Temperaturprogramm (Hauptwäsche und Stufenschleudern) unter Verwendung von 2 kg Ballaststoff durchgeführt. Die Bleichwirkung wurde als Remissionszunahme an EMPA-Teststreifen (EMPA 103) gemessen. Es wurden jeweils 4,5 g/l Waschmittel eingesetzt, die durch Mischen von IEC-Waschmittel mit dem jeweiligen Bleichsystem hergestellt wurden.

| Waschmittel | Standard | Rotwein | Blut |
|---|---|---|---|
| IEC | 24,7 | 54,8 | 65,5 |
| IEC/10 % Perborat | 27,8 | 54,9 | 37,1 |
| IEC/10 % Perborat/3 % TAED | 28,0 | 57,7 | 44,2 |
| IEC/3 % PAP | 32,2 | 57,8 | 68,5 |
| IEC/6 % PAP | 34,9 | 63,1 | 66,6 |
| IEC/9 % PAP | 34,4 | 66,6 | 65,5 |
| IEC/6 % DSIPH | 36,6 | 59,1 | 65,6 |
| TAED = Tetraacetylethylendiamin PAP = ω-Phthalimidoperoxihexansäure DSIPH = ω-[2-Dodecylsuccinimido]-peroxihexansäure | | | |

Die Versuche zeigen eine deutliche Überlegenheit der erfindungsgemäßen Imidoperoxicarbonsäure gegenüber bekannten Bleichsystemen. Insbesondere wird bei Verwendung dieser Persäuren kein antagonistischer Effekt bei der Auswaschbarkeit von Blutflecken beobachtet.

### Beispiel 14

### Bestimmung der Öllöslichkeit von Bleichsystemen

Zur Bestimmung der Öllöslichkeit eines Bleichsystems wurde das Bleichsystem bei 20°C in eine Mischung aus 50 % E-Wasser und 50 % Isopropylmyristat eingetragen, der pH-Wert auf 9 eingestellt und das Gemisch 10 min intensiv verrührt. Nach der Phasentrennung wurde der Peroxicarbonsäureanteil in Öl- und Wasserphase titrimetrisch bestimmt.

| Bleichsystem | Öllöslichkeit bei pH 9 |
|---|---|
| Perborat/TAED | 6 % |
| DPDDA | 15 % |
| PAP | 38 % |
| Perborat/Isonobs | 54 % |
| DSIPH | 86 % |

### Lagerversuche der freien Peroxicarbonsäuren

Die Versuche wurden in offenen Gefäßen im Wärmeschrank durchgeführt. Der Gehalt an Peroxicarbonsäure wurde titrimetrisch bestimmt.

| Peroxicarbonsäure | Lagerzeit (Wochen) | Temperatur | Aktivsauerstoffverlust |
|---|---|---|---|
| PAP | 4 | 25°C | 1,4 % |
| NAPSA | 4 | 25°C | 14,6 % |
| PAP | 4 | 40°C | 2,0 % |
| NAPSA | 4 | 40°C | 29,3 % |
| PAP | 4 | 50°C | 12,0 % |
| NAPSA | 2 | 50°C | 100,0 % |

## Patentansprüche

1. Verbindung der Formel

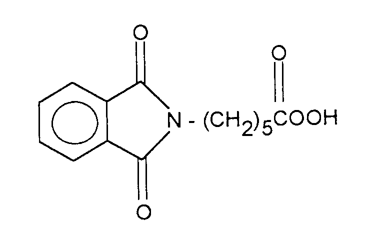

2. Verfahren zur Herstellung der Verbindung gemäß Anspruch 1, dadurch gekennzeichnet, daß man Phthalsäureanhydrid mit ω-Aminocapronsäure umsetzt und die erhaltene Imidocarbonsäure mit Wasserstoffperoxid in Gegenwart einer starken Säure oxidiert.

3. Verfahren zur Herstellung der Verbindung gemäß Anspruch 1, dadurch gekennzeichnet, daß man Phthalsäureanhydrid mit Caprolactam in Gegenwart von Wasser unter Druck umsetzt und die erhaltene Imidocarbonsäure mit Wasserstoffperoxid in Gegenwart einer starken Säure oxidiert.

4. Verfahren nach Anspruch 3, dadurch gekennzeichnet, daß die Umsetzung bei einem Überdruck von 1 bis 30 bar, vorzugsweise 2 bis 5 bar erfolgt.

5. Verfahren nach Anspruch 3 oder 4, dadurch gekennzeichnet, daß die Umsetzung über einen Zeitraum von 2 bis 20 h, vorzugsweise 5 bis 10 h, bei einer Temperatur von 100 bis 250°C, vorzugsweise 120 bis 200°C, unter Inertgasatmosphäre erfolgt.

6. Verwendung der Verbindung gemäß Anspruch 1 in Blech-, Oxidations- und Reinigungsmitteln.

## Claims

1. A compound of the formula

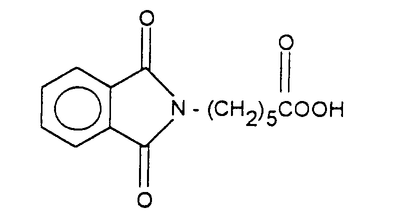

2. A process for the preparation of the compound as claimed in claim 1, which comprises reacting phthalic anhydride with ω-aminocaproic acid and oxidizing the resulting imidocarboxylic acid using hydrogen peroxide in the presence of a strong acid.

3. A process for the preparation of the compound as claimed in claim 1, which comprises reacting phthalic anhydride with caprolactam under pressure in the presence of water and oxidizing the resulting imidocarboxylic acid using hydrogen peroxide in the presence of a strong acid.

4. The process as claimed in claim 3, wherein the reaction is carried out at a gage pressure of from 1 to 30 bar, preferably from 2 to 5 bar.

5. The process as claimed in claim 3 or 4, wherein the reaction is carried out over a period of from 2 to 20 h, preferably from 5 to 10 h, at a temperature of from 100 to 250°C, preferably from 120 to 200°C, under an inert gas atmosphere.

6. The use of the compound as claimed in claim 1 in bleaching, oxidizing and cleaning compositions.

## Revendications

1. Composé de formule

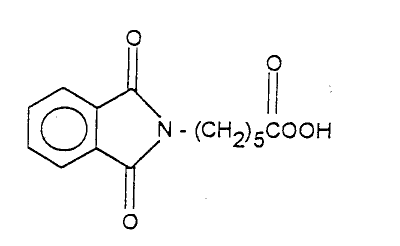

2. Procédé de préparation du composé selon la revendication 1, caractérisé en ce que l'on fait réagir l'anhydride d'acide phtalique avec l'acide ω-aminocaproïque et on oxyde l'acide imidocarboxylique obtenu avec du peroxyde d'hydrogène en présence d'un acide fort.

3. Procédé pour la préparation du composé selon la revendication 1, caractérisé en ce que l'on fait réagir l'anhydride d'acide phtalique avec le caprolactame en présence de l'eau, sous pression, et on oxyde l'acide imidocarboxylique avec du peroxyde d'hydrogène en présence d'un acide fort.

4. Procédé selon la revendication 3, caractérisé en ce que l'on effectue la réaction sous une surpression de 1 à 30 bar, de préférence de 2 à 5 bar.

5. Procédé selon la revendication 3 ou 4, caractérisé en ce que l'on effectue la réaction dans un intervalle de 2 à 20 heures, de préférence de 5 à 10 heures, à une température de 100 à 250°c, de préférence de 120 à 200 °C, sous une atmosphère de gaz inerte.

6. Utilisation du composé selon la revendication 1 dans les produits de blanchiment, d'oxydation et de nettoyage.
